# EUROPÄISCHE PATENTANMELDUNG

(11) **EP 1 415 590 A1**
(43) Veröffentlichungstag der Anmeldung: **06.05.2004**
(21) Anmeldenummer: 02024022.2
(22) Anmeldetag: 28.10.2002
(51) Int. Cl.: A61B 5/00

(54) **Glukose-Sensor**

(71) Anmelder: ECOLE POLYTECHNIQUE FEDERALE DE LAUSANNE, 1015 Lausanne (CH)
(72) Erfinder: Strässler, Sigfried, Dr., 1113 Saint-Saphorin-sur-Morges (CH); Ryser, Peter, Prof. Dr., 1110 Morges (CH); Ganz, Klaus, Dr., 8700 Küsnacht (CH); Jacot, Jaques, Prof., 2046 Fontaines (CH)
(74) Vertreter: Dittrich, Horst, Dr.

(57) **Zusammenfassung**

Der vorgeschlagene Glukose-Sensor umfasst einen durch einen Ampulle (1) gebildeten, implantierbaren Sensor und ein diesem zugeordnetes Bediengerät. In der Ampulle (1) ist eine sensitive Flüssigkeit eingeschlossen, und Glukose kann in die Ampulle (1) eindringen. Es erfolgt eine Messung der Viskosität des aus der sensitiven Flüssigkeit und der Glukose bestehenden Gemisches. Das Bediengerät, welches die Messung und deren Auswertung steuert, ist durch ein aussen auf der Haut zu tragendes, portables Gerät gebildet. Die Messung der Viskosität erfolgt anhand des Schwingverhaltens eines in der Ampulle (1) angeordneten Schwingorgans (8), welches von einem ebenfalls in der Ampulle (1) angeordneten Magneten (6) zu Schwingungen anregbar ist. Das Schwingverhalten wird anhand des Abklingverhaltens des Schwingorgans (8) nach Abschalten des Magneten (6) analysiert.

## Beschreibung

Die Erfindung liegt auf dem Gebiet der Bestimmung der Glukose-Konzentration im Blut, die für die Behandlung von Diabetes von grosser Wichtigkeit ist. Bekanntlich ist bei Diabetikern die körpereigene Produktion von Insulin, welches für die Abläufe des Glukosestoffwechsels im Körper eine grosse Rolle spielt, entweder nicht mehr ausreichend oder ganz eingestellt worden. Diabetes ist durch eine chronische Störung des gesamten Fett- und Kohlehydrat-Stoffwechsels gekennzeichnet, welche unter anderem zu Bluthochdruck und erhöhten Blutfettwerten führt, welche beide die Entstehung von Arteriosklerose begünstigen. Dabei verengen sich die Blutgefässe mit den möglichen Folgen von Herzinfarkt, Schlaganfall, Nierenversagen und Augenschäden bis zur Erblindung. Zudem führen Nervenschädigungen zu Taubheitserscheinungen in den Gliedmassen, so dass viele Patienten im fortgeschrittenen Stadium der Erkrankung Verletzungen ihrer Füsse nicht mehr spüren. Zusammen mit der Gefässverengung entwickelt sich auf diese Weise der so genannte diabetische Fuss, die weitaus häufigste, nicht unfallbedingte Ursache von Amputationen.

Aus den geschilderten Gründen hat heute Diabetes in allen Industriestaaten einen grossen Einfluss auf die Gesundheitskosten. Laut der Welt-Gesundheits-Organisation WHO benötigen heute weltweite etwa 130 Millionen Menschen eine Behandlung gegen Diabetes, wobei die Tendenz steigend ist. Innerhalb der nächsten 25 Jahre wird ein Anstieg auf 300 Millionen Menschen geschätzt. Die in den USA von Diabetes-Erkrankungen verursachten Kosten wurden im Jahr 1997 auf 98 Milliarden USD geschätzt.

Zur Vermeidung von irreparablen gesundheitlichen Folgeschäden ist eine möglichst genaue Einstellung der Blutkonzentration mittels Insulinzufuhr notwendig, wobei es für die Bestimmung der erforderlichen Insulinmenge erforderlich ist, den Glukosegehalt im Blut zu kennen. Die Blutzuckerkonzentrationsmessung wird heute mittels einer Miniblutprobe bestimmt, was für den Patienten mit Unannehmlichkeiten verbunden ist. Aus diesen Gründen wird schon seit einiger Zeit ein schmerzfreies, möglichst kontinuierliches Behandlungsverfahren angestrebt, wodurch die Folgekosten der Krankheit gesenkt und die Lebensqualität der von Diabetes betroffenen Menschen erhöht würde.

Die vorliegende Erfindung betrifft ein Sensorsystem für die Bestimmung der Glukose-Konzentration im Blut, mit einem implantierbaren Sensor und einem diesem zugeordneten Bediengerät.

Es ist ein transcutanes System mit einem implantierbaren Sensor bekannt, der eine Nadel aufweist, welche zwei durch einen Isolator getrennte verschiedene Metalle enthält, so dass ein elektrisches Potential angelegt werden kann. Der Sensor ist mit einem Monitor verbunden, der alle 5 Minuten über maximal 3 Tage die Glukosewerte aufzeichnet. Der Sensor ist nicht sehr stabil, so dass mehrmals täglich eine Eichung mit dem Blut des Patienten vorgenommen werden muss.

Bei einem anderen heute auf dem Markt erhältlichen Messsystem zur Messung des Glukosegehalts wird durch Stromimpulse Glukose durch die Haut gezogen und in zwei Gel-Scheiben eines Sensors gesammelt, welcher den Glukosegehalt misst. Der Sensor, der auf der Rückseite eines uhrartigen Anzeigegeräts angeordnet ist, ist ein so genanntes minimal invasives System, das ist ein System, bei welchem man entweder etwas auf die Haut auftragen oder kleine Kanülen in diese stechen muss, wodurch ein Infektionsrisiko nicht ausgeschlossen werden kann. Aus diesem Grund muss bei diesem invasiven System der Sensor alle paar Tage gewechselt werden, ausserdem erfordert auch dieses System eine Eichung mit dem Blut des Patienten. Beide genannten bekannten Systeme werden auch als Holtersysteme bezeichnet, darunter versteht man Systeme für den Gebrauch durch einen Arzt und nicht durch den Patienten selbst.

Durch die Erfindung soll ein Sensorsystem angegeben werden, welches für den Gebrauch durch den Patienten geeignet ist und diesem eine ständige Überwachung des Glukosegehalts seines Blutes ermöglichen soll, ohne dass nach der Implantation des Sensors schon nach kurzer Zeit ein neuerlicher Eingriff erforderlich ist, oder ein Infektionsrisiko darstellende Manipulationen an oder in der Haut des Patienten erforderlich sind.

Die gestellte Aufgabe wird erfindungsgemäss dadurch gelöst, dass der Sensor durch eine Ampulle gebildet ist, in welcher eine sensitive Flüssigkeit eingeschlossen ist und in welche Glukose eindringen kann, dass eine Messung der Viskosität des aus der sensitiven Flüssigkeit und der Glukose bestehenden Gemisches erfolgt, und dass das Bediengerät durch ein aussen auf der Haut zu tragendes, portables Gerät gebildet ist, wobei die Steuerung der Messung und deren Auswertung durch das Bediengerät erfolgt.

Beim erfindungsgemässen Sensorsystem erfolgt durch das Bediengerät keinerlei Manipulation an oder in der Haut, so dass jede Reiz- und Infektionsgefahr ausgeschlossen ist. Der Sensor kann zumindest mehrere Monate lang implantiert sein, ohne dass eine Nacheichung oder dergleichen erforderlich wäre und dem Patienten bleiben die lästigen Blutabnahmen erspart. Der Patient kann jederzeit ohne irgendwelche Beschwerden den Glukosegehalt seines Blutes prüfen und diesen durch Einnahme entsprechender Medikamente regulieren, ohne dass eine Überwachung durch einen Arzt erforderlich wäre.

Eine erste bevorzugte Ausführungsform des erfindungsgemässen Sensorsystems ist dadurch gekennzeichnet, dass die Messung der Viskosität anhand des Schwingverhaltens eines in der Ampulle angeordneten Schwingorgans erfolgt, welches von einem ebenfalls in der Ampulle angeordneten Magneten zu Schwingungen anregbar ist. Das Schwingverhalten des Schwingorgans wird anhand von dessen Abklingverhalten nach der Anregung analysiert.

Eine zweite bevorzugte Ausführungsform des erfindungsgemässen Sensorsystems ist dadurch gekennzeichnet, dass durch das Schwingorgan zusätzliche eine Homogenisierung der Flüssigkeit in der Ampulle erfolgt.

Eine dritte bevorzugte Ausführungsform des erfindungsgemässen Sensorsystems ist dadurch gekennzeichnet, dass das Schwingorgan formschlüssig mit dem Magneten verbunden und durch einen Biegebalken gebildet ist.

Eine vierte bevorzugte Ausführungsform ist dadurch gekennzeichnet, dass der Magnet an einem der beiden Enden des Biegebalkens angebracht und durch ein Magnetfeld in Schwingungen versetzbar ist. Dieses Magnetfeld wird entweder durch eine im Bediengerät vorgesehene elektromagnetische Anordnung oder durch eine in der Ampulle vorgesehene elektrische Spule erzeugt.

Eine fünfte bevorzugte Ausführungsform des erfindungsgemässen Sensorsystems ist dadurch gekennzeichnet, dass die Ampulle eine das Eindringen von Glukose ermöglichende semipermeable Wand aufweist.

Eine weitere bevorzugte Ausführungsform ist dadurch gekennzeichnet, dass in der Ampulle eine diese teilweise ausfüllendes und damit das Flüssigkeitsvolumen begrenzendes Kunststoffteil angeordnet ist. Das Kunststoffteil ist vorzugsweise als Auflage für das Schwingorgan ausgebildet und weist eine längliche Bohrung auf, in welche ein am Magneten angeordneter und zur Durchmischung der Flüssigkeiten vorgesehener Flügel ragt.

Im Folgenden wird die Erfindung anhand eines Ausführungsbeispiels und der Zeichnungen näher erläutert; es zeigt:
- Fig. 1, 2: je eine perspektivische Darstellung des teilweise aufgeschnittenen Sensors einer erfindungsgemässen Einrichtung,
- Fig. 3: einen Querschnitt durch den Sensor von Fig. 1, 2; und
- Fig. 4: ein Blockschema des Bediengeräts der erfindungsgemässen Einrichtung.

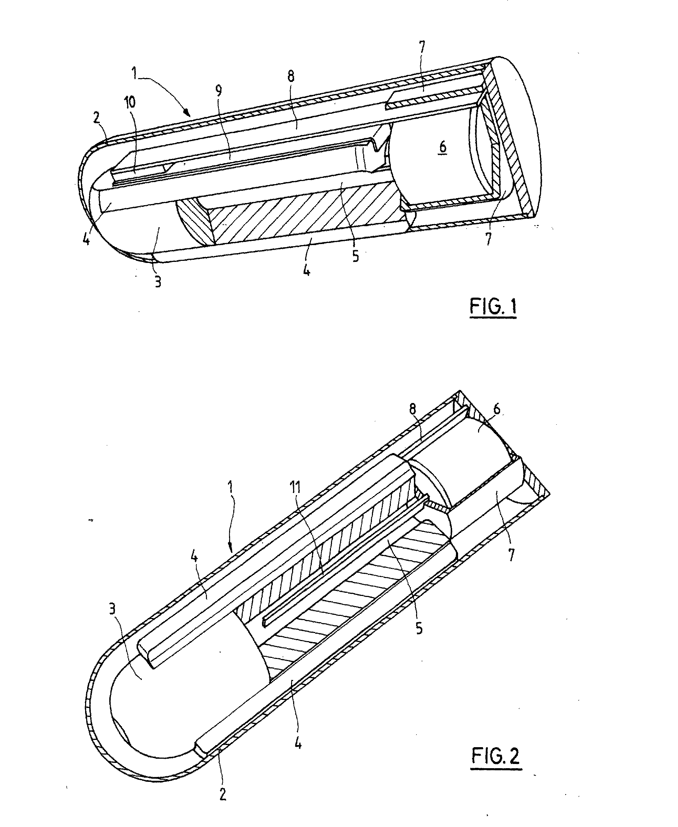

Der in den Figuren 1 bis 3 dargestellte Sensor hat die Form einer länglichen Ampulle 1 mit den ungefähren Dimensionen von 2 mm Durchmesser und 8 mm Länge, wobei diese Angaben in weiten Grenzen variabel sind. Der Mantel 2 der Ampulle ist durch eine semipermeable Wand aus Cellulose gebildet, durch welche Glukose in die Ampulle eindringen kann. Der grösste Teil des Innenraums der Ampulle 1 wird von einem zylindrischen Kunststoffteil 3 eingenommen, das durch mehrere an seinem Mantel abstehende Rippen 4 in der Ampulle 1 zentriert ist und eine axiale Bohrung 5 aufweist. Das Kunststoffteil, das beispielsweise ein aus Polycarbonat hergestelltes Spritzgussteil ist, dient einerseits als Träger für ein weiter unten beschriebenes Schwingorgan und anderseits zur Verkleinerung des Flüssigkeitsvolumens in der Ampulle 1. Die Ampulle ist im betriebsbereiten Zustand mit einer sensitiven Flüssigkeit mit hohem Molekulargewicht, beispielsweise Dextran und ConA gefüllt.

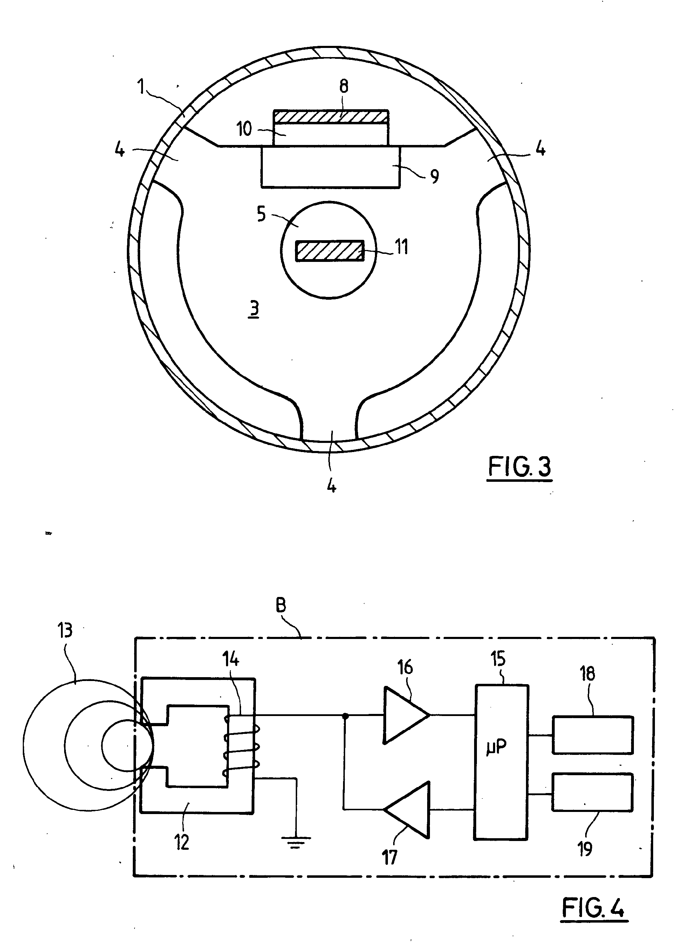

Im Anschluss an das in den Fig. 1 und 2 rechte Ende des Kunststoffteils 3 ist in der Ampulle 1 ein Permanentmagnet 6 angeordnet, der zur Vermeidung von Korrosion mit einer Kunststoffummantelung 7 aus Polycarbonat überspritzt ist. In den Fig. 1 und 2 sind die Kunststoffummantelung 7, der Mantel 2 der Ampulle 1 und das Kunststoffteil 3 teilweise aufgeschnitten, um einen Blick ins Innere des Kunststoffteils 3 zu ermöglichen. Die Kunststoffummantelung 7 dient als Träger für einen Biegebalken 8 aus beispielsweise Aluminiumoxidkeramik, der sich entlang des Kunststoffteils 3 erstreckt. Das Kunststoffteil 3 ist im Bereich des Biegebalkens 8 abgeflacht (Fig. 3) und trägt hier ein Basissubstrat 9 in Form eines dünnen Streifens. Zwischen dem freien Ende des Biegebalkens 8 und dem Basissubstrat 9 ist ein Distanzelement 10 vorgesehen, dessen Dicke so gewählt ist, dass eine genügend grosse Schwingungsamplitude des Biegebalkens von etwa 100 µm möglich ist.

Biegebalken 8, Basissubstrat 9 und Distanzelement 10 bestehen aus dem gleichen Material und sind durch aufeinander Schichten von Laminaten und anschliessendes Verpacken hergestellt. Die Kunststoffummantelung 7 trägt an ihrer dem Kunststoffteil 3 zugewandten Stirnfläche einen schmalen., langgestreckten Flügel 11, der in die Bohrung 5 des Kunststoffteils 3 ragt. Wenn der Permanentmagnet 6 von einem externen oszillierenden Magnetfeld erregt wird, wird er in Vibrationen versetzt und mit der Vibration des Magneten 6 vibrieren auch die Kunststoffummantelung 7, der Biegebalken 8 und der Flügel 11. Diese Vibrationen haben zur Folge, dass die in der Ampulle 1 vorhandene sensitive Flüssigkeit mit der in die Ampulle 1 eingedrungenen Glukose vermischt wird. Dabei ist die Vibration des Flügels 11 von grosser Bedeutung für eine schnelle Messung, weil sie den Fluss in der Ampulle 1 simuliert und für eine homogene Glukosekonzentration in der Ampulle sorgt.

Die Frequenz des den Magneten 6 erregenden Magnetfeldes ist so gewählt, dass dieser mit einer Frequenz im Bereich zwischen 100 und 300 Hz vibriert. Biegebalken 8 und Flügel 11 vibrieren mit der gleichen Frequenz, wobei die Schwingungsamplitude etwa 100 µm oder 0.1 mm beträgt. Nach der Durchmischung von sensitiver Flüssigkeit und Glukose wird das Magnetfeld abgeschaltet und die Abklingzeit der Vibration gemessen, was anhand des von dem mit dem Biegebalken 8 mit schwingenden Magneten 6 erzeugten Magnetfeldes erfolgt.

Die Viskositätsänderung von Dextran und ConA in einer physiologisch salinen Lösung in Funktion der Glukose-Konzentration ist in R. Ehwald et al., "Viscosimetric affinity assay", Anal Biochem 234,1 (1996) und U. Beyer, "Recording of subcutaneous glucose dynamics by a viscosimetric affinity sensor", Diabetologia 44, 416 (2001) beschrieben. Die dort beschriebene Lösung basiert auf der Zirkulation der sensitiven Flüssigkeit durch ein aus mehreren Komponenten bestehendes System. Beim erfindungsgemässen System wird die Viskosität direkt im Volumen der in der Ampulle 1 eingeschlossenen sensitiven Flüssigkeit gemessen, wobei die Ampulle in Längsrichtung senkrecht zur Körperoberfläche so unter die Haut implantiert wird, dass das in den Fig. 1 und 2 flache rechte Ende der Ampulle etwa 2 mm unterhalb der Haut liegt. Die Implantation erfolgt beispielsweise in Gürtelhöhe mit einer Injektionsnadel.

Fig. 4 zeigt ein Blockschema des mit dem Bezugszeichen B bezeichneten Bediengeräts. Dieses enthält insbesondere einen Magneten 12 zur Erzeugung eines Magnetfeldes 13 für die Erregung des Magneten 6 in der Ampulle 1 (Fig. 1) und eine Spule 13 für die Erregung des Magneten 12, welche gleichzeitig auch als Magnetfeldsensor für die Detektion des vom Magneten 6 in der Ampulle 1 erzeugten Magnetfeldes dienen, und einen Mikroprozessor 15. Die Spule 14 ist einerseits mit einem Empfangsverstärker 16 und andererseits mit einem Sendeverstärker 17 verbunden, deren Aus- bzw. Eingang an den Mikroprozessor 15 geführt ist. Der Mikroprozessor 15 ist ausserdem mit einer Anzeige 18 für den aktuell gemessenen Glukosewert und mit einem Speicher 19 für die Speicherung der Glukosewerte verbunden. Ausserdem enthält das Bediengerät B eine nicht eingezeichnete Stromversorgung. Optional kann ein zusätzlicher Magnetfeldsensor, beispielsweise ein Hall-Sensor, für die genaue Positionierung (Normalisierung) des Bediengeräts B relativ zur Ampulle 1 vorgesehen sein.

Eine andere mögliche Lösung für die Funktionen Anregung und Detektion des Bediengeräts B beruht auf einem rotierenden Dipol, wobei ein Harddisk-Motor mit zwei Permanentmagneten die Schwingungen des Biegebalkens 8 von aussen anregt und durch Analyse der Motordämpfung der Gütefaktor des Oszillators (Biegebalken 8 plus Magnet 6) ermittelt wird.

Das beschriebene Sensorsystem kann durch relativ einfache Modifikation für die Anwendung als Holtersystem adaptiert werden, bei welchem der Glukosegehalt unter ärztlicher Aufsicht und über einen Zeitraum von mehreren Tagen kontinuierlich überwacht wird. Bei dieser Anwendung wird das den Magneten 6 in der Ampulle 1 erregende Magnetfeld nicht von einem externen Magneten 12 sondern durch eine im Inneren der Ampulle 1 angeordnete Stromspule erzeugt, von welcher zwei dünne elektrische Drähte durch die Haut des Patienten nach aussen zum Bediengerät geführt sind. Die genannte Stromspule ist vorzugsweise im Bereich des Distanzelements 10 (Fig. 1) angeordnet. Um einen ausreichenden Magnetfluss von der Stromspule zum Magneten 6 zu gewährleisten, bestehen Biegebalken 8 und Basissubstrat 9 aus weichmagnetischem Material.

## Patentansprüche

1. Sensorsystem für die Bestimmung der Glukose-Konzentration im Blut, mit einem implantierbaren Sensor und einem diesem zugeordneten Bediengerät (B), **dadurch gekennzeichnet dass** der Sensor durch eine Ampulle (1) gebildet ist, in welcher eine sensitive Flüssigkeit eingeschlossen ist und in welche Glukose eindringen kann, dass eine Messung der Viskosität des aus der sensitiven Flüssigkeit und der Glukose bestehenden Gemisches erfolgt, und dass das Bediengerät (B) durch ein aussen auf der Haut zu tragendes, portables Gerät gebildet ist, wobei die Steuerung der Messung und deren Auswertung durch das Bediengerät (B) erfolgt.

2. Sensorsystem nach Anspruch 1, **dadurch gekennzeichnet, dass** die Messung der Viskosität anhand des Schwingverhaltens eines in der Ampulle (1) angeordneten Schwingorgans (8) erfolgt, welches von einem ebenfalls in der Ampulle (1) angeordneten Magneten (6) zu Schwingungen anregbar ist.

3. Sensorsystem nach Anspruch 2, **dadurch gekennzeichnet, dass** das Schwingverhalten des Schwingorgans (8) anhand von dessen Abklingverhalten nach Abschalten des Magneten (6) analysiert wird.

4. Sensorsystem nach Anspruch 2 oder 3, **dadurch gekennzeichnet, dass** durch das Schwingorgan zusätzlich eine Homogenisierung der Flüssigkeit in der Ampulle (1) erfolgt.

5. Sensorsystem nach Anspruch 4, **dadurch gekennzeichnet, dass** das Schwingorgan (8) formschlüssig mit dem Magneten (6) verbunden und durch einen Biegebalken gebildet ist.

6. Sensorsystem nach Anspruch 5, **dadurch gekennzeichnet, dass** der Magnet (6) an einem der beiden Enden des Biegebalkens angebracht und durch ein Magnetfeld (13) in Schwingungen versetzbar ist.

7. Sensorsystem nach Anspruch 6, **dadurch gekennzeichnet, dass** das genannte Magnetfeld (13) durch eine im Bediengerät (B) vorgesehene elektromagnetische Anordnung erzeugt wird.

8. Sensorsystem nach Anspruch 6, **dadurch gekennzeichnet, dass** das genannte Magnetfeld durch eine in der Ampulle (1) vorgesehene elektrische Spule erzeugt wird.

9. Sensorsystem nach einem der Ansprüche 2 bis 8, **dadurch gekennzeichnet, dass** die Ampulle (1) eine das Eindringen von Glukose ermöglichende semipermeable Wand (2) aufweist.

10. Sensorsystem nach einem der Ansprüche 2 bis 9, **dadurch gekennzeichnet, dass** in der Ampulle (1) ein diese teilweise ausfüllendes und damit das Flüssigkeitsvolumen begrenzendes Kunststoffteil (3) angeordnet ist.

11. Sensorsystem nach Anspruch 10, **dadurch gekennzeichnet, dass** das Kunststoffteil (3) als Auflage für das Schwingorgan (8) ausgebildet ist.

12. Sensorsystem nach Anspruch 10 oder 11, **dadurch gekennzeichnet, dass** das Kunststoffteil (3) eine längliche Bohrung (5) aufweist, in welche ein am Magneten (6) angeordneter und zur Durchmischung der Flüssigkeiten vorgesehener Flügel (11) ragt.

13. Sensorsystem nach Anspruch 7, **dadurch gekennzeichnet, dass** die genannte elektromagnetische Anordnung Mittel zur Erregung des Magneten (6) in der Ampulle (1) und einen Sensor für das von diesem Magneten erzeugte Magnetfeld enthält.

14. Sensorsystem nach Anspruch 13, **dadurch gekennzeichnet, dass** die genannten Mittel und der genannte Sensor durch einen Magneten (12) und eine diesen erregende Spule (14) sowie einen mit der Spule (14) verbundenen Mikroprozessor (15) gebildet sind.
